Europäisches Patentamt

European Patent Office

Office européen des brevets

(11) Veröffentlichungsnummer: **0 385 268**
**A2**

## (12) EUROPÄISCHE PATENTANMELDUNG

(21) Anmeldenummer: **90103437.1**

(51) Int. Cl.5: **A61M 25/00**

(22) Anmeldetag: **22.02.90**

(30) Priorität: **27.02.89 DE 3906027**

(43) Veröffentlichungstag der Anmeldung:
**05.09.90 Patentblatt 90/36**

(84) Benannte Vertragsstaaten:
**DE ES FR GB IT**

(71) Anmelder: **Fresenius AG**
**Gluckensteinweg 5**
**D-6380 Bad Homburg v.d.H.(DE)**

(72) Erfinder: **Krick, Gerd, Dr. Dipl.-Ing.**
**Kaiser-Friedrich-Promenade 92**
**D-6380 Bad Homburg v.d.H.(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Schlauch zur Führung von medizinischen Flüssigkeiten.**

(57) Die Erfindung betrifft einen Schlauch (1) zur Führung von medizinischen Flüssigkeiten. Bei bekannten Schläuchen, insbesondere aus weichmacherfreien Materialien hat es sich als nachteilig erwiesen, daß die Schläuche eine geringe Festigkeit aufweisen und leicht geknickt oder gequetscht werden können. Zur Verstärkung des Schlauches (1) ist erfindungsgemäß vorgesehen, daß bestimmte Wandungsbereiche (2) eine größere Dicke aufweisen, welche sich im wesentlichen ringförmig um den Schlauch (1) erstrecken. Der Schlauch (1) ist erfindungsgemäß wesentlich verstärkt, wobei durch die besondere Ausbildung des Schlauches (1) eine Verstärkung auch bei sehr kleinen Schlauchdurchmessern erfolgen kann. Weiterhin ist der erfindungsgemäße Schlauch (1) kostengünstig herstellbar.

FIG.1

EP 0 385 268 A2

## Schlauch zur Führung von medizinischen Flüssigkeiten

Die Erfindung bezieht sich auf einen Schlauch zur Führung von medizinischen Flüssigkeiten.

Für Schläuche zur Führung medizinischer Flüssigkeiten gibt es vielfältige Anwendungsfälle, beispielsweise dienen derartige Schläuche dem Blut- oder Plasmatransport oder zur Einleitung von Infusionslösungen.

Die bisher aus dem Stand der Technik bekannten Schläuche bestehen üblicherweise aus einem PVC-Material, welches den erheblichen Nachteil aufweist, daß es Weichmacher enthält, welche während der Verwendung des Schlauches ausgelaugt oder ausdiffundiert werden können, so daß die Gefahr besteht, daß die Weichmachersubstanzen in die medizinische Flüssigkeit gelangen. Aus diesem Grunde wurde in der Vergangenheit versucht, weichmacherfreie Materialien für derartige Schläuche zu verwenden. Bei den dafür zur Verfügung stehenden Materalien, z. B. PE, PP, EVA erweist es sich jedoch als nachteilig, daß das Schlauchmaterial relativ weich ist und nur eine sehr geringe Rückprallelastizität aufweist, so daß die Rückstellkraft des Materials nicht ausreicht, nach einer Pressung oder Quetschung des Schlauches diesen wieder zu seinem Ursprungsquerschnitt zurückzuverformen. Daraus ergibt sich der Nachteil, daß der Schlauch an den gepreßten oder gedrückten Stellen in seinem Querschnitt verengt bleibt und eine freie Durchströmung deshalb verhindert wird. Dies erweist sich insbesondere dann als besonders nachteilig, wenn die medizinischen Flüssigkeiten drucklos oder unter sehr geringem Druck durch den Schlauch geführt werden sollen, beispielsweise bei Infusionen. Es wurde versucht, diesen Nachteil durch eine größere Wanddicke des Schlauches auszugleichen, dies erwies sich jedoch als nachteilig, da derartige Schläuche dann insgesamt sehr unflexibel und schlecht handhabbar sind.

Die oben stehende Problematik wird bei Schläuchen zur Führung medizinischer Flüssigkeiten insbesondere dadurch verschärft, daß die Schläuche einen relativ geringen Innendurchmesser aufweisen, so daß bereits geringste Kräfte ausreichen, den Schlauch abzuknicken oder zu quetschen und den Durchfluß zu unterbinden.

Es ist aus dem Stand der Technik bekannt, die Festigkeit von Schläuchen dadurch zu verstärken, daß in den Schlauch eine Spirale aus einem metallischen Drahtwerkstoff eingebettet wird. Ein derartiger Schlauch ist beispielsweise aus der US-PS 243396 oder aus dem DE-GBM 8033825 bekannt. Diese Ausgestaltung eines Schlauches ist jedoch für die hier zur Rede stehenden Anwendungsfälle nicht geeignet, da es erforderlich ist, daß der Schlauch einen bestimmten Mindestdurchmesser aufweist, um überhaupt eine metallische Spirale mit dem Schlauch verbinden zu können. Weiterhin ist es erforderlich, daß die Wanddicke des Schlauches ein bestimmtes Maß aufweist, um die metallische Drahtspirale in die Wandung des Schlauches einzubetten. Aus alldem ergibt sich, daß derartige Schläuche erst ab einem relativ großen Querschnittsbereich technisch realisierbar sind und somit zur Führung von medizinischen Flüssigkeiten ausscheiden. Ein weiterer Nachteil dieser Schläuche liegt darin, daß die Herstellungskosten sehr hoch sind, so daß auch aus wirtschaftlichen Gründen eine Anwendung eines derartigen Schlauches als Einwegartikel ausscheidet.

Ein Schlauch der im Oberbegriff des Anspruches 1 angegebenen Art ist aus der DE-AS 11 20 682 bekannt. Dieser bekannte Schlauch weist einen wendelförmig gewundenen Draht auf, der von einer Hülle umgeben ist. In Spalte 6, Zeilen 47 bis 61 der genannten Druckschrift wird ausgeführt, daß die äußere wellenförmig ausgebildete Oberfläche mit Erhöhungen und Vertiefungen dazu führt, daß die Biegsamkeit des Erzeugnisse erhöht wird. Somit werden bei dem gattungsgemäßen Schlauch die verdickten Wandbereiche nicht zur Verstärkung sondern zur Erhöhung der Biegsamkeit vorgesehen.

Aus der DE-AS 29 46 385 ist ferner ein Kunststoffschlauch bekannt, bei dem zur Erhöhung der Verwindungssteifigkeit zumindest eine sogenannte Wendel vorgesehen ist, die sich über die ganze Länge des Schlauches erstreckt und die aus einem Kunststoff besteht, der in der Längsrichtung der Wendel eine hohe und quer dazu eine niedrige Zugfestigkeit besitzt. Gemäß den Ausführungen in den einleitenden Absätzen der Figurenbeschreibung handelt es sich bei der genannten Wendel um eine Kunststoffband, das außen um den Schlauch herumgewickelt wird. Hierbei ist es zur Erhöhung der Flexibilität bzw. Biegsamkeit des Schlauches möglich, einen Spalt vorzusehen.

Die DE-AS 29 38 943 beschreibt ein Verstärkungselement für Schläuche oder Bälge, das aus einem Ring aus hohlem profiliertem Material besteht, innerhalb dem ein Ring verklemmt ist, und bei dem das Schlauchmaterial zwischen den beiden zuvor genannten Ringen eingeklemmt ist. Diese Art der Verstärkung führt beispielsweise bei der Ausführungsform gemäß der genannten Druckschrift zu dem Nachteil, daß die Innenwandung des Schlauches nicht mehr glattflächig ist, die an sich selbst nicht verdickt ist, sondern durch ein von außen aufzubringendes Klemmteil verstärkt wird. Weiterhin ist aus der DE-AS 32 39 475 ein Schlauch für medizinische Geräte bekannt, bei dem Rippen vorgesehen sind, welche jedoch lediglich die Neigung zu einem Zusammenkleben bei der autoklaven Sterilisierung herabsetzen sollen. Die Rippen könnten hierbei entweder in Längsrichtung des Schlauches oder in langgestreckten Wendeln angeordnet sein.

Aufgrund der Ausbildung und Ausrichtung der bekannten Rippen dieses bekannten Schlauches sind diese jedoch nicht dazu geeignet, gleichzeitig eine hohe Flexibilität und einen ausreichenden Widerstand gegen Druchpressen oder Quetschen hervorgerufene Querschnittsverringerungen aufzubauen.

Weiterhin sind aus dem Stand der Technik Schläuche bekannt, welche eine Textilverstärkung aufweisen, welche in die Wandung des Schlauches eingebettet ist, so wie dies beispielsweise auf dem Bereiche der Gartenschläuche bekannt ist.

Auch diese Schläuche sind in der Herstellung aufwendig und erfordern einen relativ großen Mindestdurchmesser, um die Art der Herstellung und Verstärkung überhaupt anwenden zu können.

Der Erfindung liegt die Aufgabe zugrunde, einen Schlauch zur Führung von medizinischen Flüssigkeiten zu schaffen, welcher bei einfachem Aufbau und betriebssicherer Anwendbarkeit eine hohe Flexibilität aufweist und gegen durch Pressen oder Quetschen hervorgerufene Querschnittsverringerungen einen ausreichenden Widerstand entgegensetzt.

Erfindungsgemäß wird diese Aufgabe dadurch gelöst, daß der Schlauch Wandungsbereiche größerer Dicke aufweist, welche sich im wesentlichen ringförmig um den Schlauch erstrecken, wobei die Wandungsbereiche eine Dicke aufweisen, welche um einen Faktor 2 bis 10 größer ist als die Dicke der nicht-verdickten Schlauchwandung.

Der erfindungsgemäße Schlauch zeichnet sich durch eine Reihe erheblicher Vorteile aus.

Da der Schlauch, zusätzlich zu der normalen Wandungsdicke Wandungsbereiche größerer Dicke aufweist, d. h. in einzelnen Bereichen verstärkt ist, wird ohne Verwendung zusätzlicher Elemente, wie Drahtspiralen oder ähnlichem ein hoher Verstärkungseffekt erzielt.

Der erfindungsgemäße Schlauch ist sehr stabil und läßt sich nur unter erheblichem Kraftaufwand quetschen oder zusammenpressen.

Da die Stabilität des Schlauches durch die Wandungsbereiche größerer Dicke hervorgerufen wird, ist es möglich, durch geeignete Dimensionierung der Dicke dieser Wandungsbereiche eine ausreichende Festigkeit des Schlauches zu gewährleisten ohne daß der restliche Wandungsbereich des Schlauches mit einer größeren Dicke ausgebildet werden müßte.

Der Schlauch als solcher bleibt somit sehr flexibel und duktil und kann somit problemlos für die geforderten Anwendungsfälle, beispielsweise zur Förderung von Blut oder Plasma oder zur Einleitung von Infusionen versendet werden. Es liegt dabei keine Steifigkeit des Schlauches als solche vor, welche bei der medizinischen Verwendung hinderlich wäre, beispielsweise eine Führung des Schlauches parallel zur Körperoberfläche des Patienten behindern würde. Ein weiterer Vorteil des erfindungsgemäßen Schlauches liegt darin, daß die Verstärkung durch die verdickten Wandbereiche im wesentlichen ringförmig erfolgt, so daß der Schlauch gegen ein Drücken oder Pressen beständig ist, die Biegsamkeit des Schlauches als solche jedoch nicht beeinträchtigt wird, so daß es durch entsprechende Wahl des Abstandes und der Breite der verdickten Wandungsbereiche möglich ist, den Schlauch den jeweiligen Anwendungsfällen optimal anzupassen.

In besonders günstiger Ausgestaltung ist der erfindungsgemäße Ring so ausgebildet, daß die verdickten Wandungsbereiche in Form konzentrischer Ringe oder in Form einer Spirale ausgestaltet sind. In beiden Fällen wird erreicht, daß der Schlauch bei Aufrechterhaltung eines hohen Maßes an Flexibilität in ausreichender Weise versteift ist.

Ein weiterer, besonders wesentlicher Vorteil des erfindungsgemäßen Schlauches liegt darin, daß der Schlauch eine glatte, zylindrische Innenfläche aufweist. Die Durchleitung der medizinischen Flüssigkeit wird somit nicht beeinträchtigt, es ist beispielsweise möglich, diese in laminarer Strömung durch den Schlauch zu führen. Das Auftreten von Turbulenzen, welche bei dünnwandigen, mittels einer Drahtspirale verstärkten Schläuchen beobachten werden, wird somit vermieden. Insgesamt bietet der erfindungsgemäße Schlauch somit ein hohes Maß an Betriebssicherheit, da durch die Vermeidung turbulenter Strömungsverhältnisse insbesondere auch das Auftreten von Gas- oder Luftblasen unterdrückt werden, welche in medizinischen Flüssigkeiten nicht erwünscht sind. Um sowohl eine einfache Herstellbarkeit des Schlauches zu ermöglichen als auch dessen oben beschriebene Vorteile hin sichtlich seiner Anwendung besonders nachhaltig ausprägen zu können, ist erfindungsgemäß vorgesehen, daß die Wandungsbereiche eine Dicke aufweisen, welche um einen Faktor 2 - 10 größer ist, als die Dicke der nicht verdickten Schlauchwandung. Es ist somit möglich, den eigentlichen Schlauch im Extremfall als Folienschlauch auszubilden, welcher mit relativ dicken, ringförmigen Wandungsbereichen, die an der Außenseite des Schlauches angeordnet sind, verstärkt ist.

Falls erfindungsgemäß die Wandungsbereiche in Form einer Spirale ausgebildet sind, ist es günstig, die Steigungshöhe der Spirale kleiner oder gleich dem Durchmesser des Schlauches zu wählen, da dann sowohl eine ausreichende Festigkeit als auch eine gute Duktilität und Biegbarkeit des Schlauches gegeben ist.

Bei der Herstellung des erfindungsgemäßen Schlauches erweist es sich als günstig, wenn die verdickten Wandungsbereiche aus dem gleichen Material wie der Schlauch gefertigt sind, es ist jedoch auch möglich, diese aus einem unterschiedlichen Material herzustellen. So ist es insbesondere möglich, den Schlauch selbst aus einem weichmacherfreien Material zu fertigen, während das Material für die verdickten Wandungsbereiche, da es mit der medizinischen Flüssigkeit nicht in Kontakt kommt, Weichmacher enthalten kann. Es ist auch möglich, das Material für die verdickten Wandungsbereiche einzufärben, während der Schlauch selbst transparent ausgebildet ist. Bei dieser Ausgestaltung ist zum einen eine Überwachung der Durchströmung des Schlauches möglich, zum anderen können verschiedenfarbig eingefärbte Schläuche den Bedienungskomfort und die Betriebssicherheit erheblich erhöhen, da Verwechslungen mehrerer Schläuche, z. B. beim Aufbau einer Apparatur, vermieden werden können.

Wie oben stehend bereits ausgeführt, wird der erfindungs gemäße Schlauch bevorzugterweise aus einem thermoplastischen Material gefertigt, welches bevorzugterweise keine Weichmacher aufweist. Es ist jedoch für andere Anwendungsfälle auch möglich, den Schlauch aus beliebigen elastisch verformbaren Materialien herzustellen.

Der erfindungsgemäße Schlauch kann unter Verwendung eines erfindungsgemäßen Verfahrens dadurch hergestellt werden, daß der Schlauch mittels eines Extruders unter gleichzeitiger Ausbilung der verdickten Wandbereiche hergestellt wird. Dabei ist es möglich, eine Spiralige Ausbildung der wandbereiche beispielsweise durch eine Drehung des Extruderkopfes zu verwirklichen. Es ist jedoch auch möglich, den Schlauch im Bereich des Extruders oder nachfolgend an den Extruderkopf mittels eines Presswerkzeuges so zu bearbeiten, daß dessen Aussenfläche konzentrische verdickte Ringe aufweist.

Alternativ dazu ist es erfindungsgemäß auch möglich, auf einen bereits hergestellten Schlauch gleichbleibender Wanddicke die verdickten Wandungsbereiche aufzubringen, beispielsweise durch Auflegen und Verschweißen oder Verkleben eines bandförmigen Kunststoffmaterials.

Die Verstärkung des Wandbereichs des erfindungsgemäßen Schlauches weist zusätzlich zu der Knick- und Druckfestigkeit den Vorteil auf, daß es möglich ist, diesen Schlauch, welcher im Extrem als Folienschlauch ausgebildet sein kann, mit einem Unterdruck zu beaufschlagen, beispielsweise einem Unterdruck von 250 mm Hg. Die verdickten Wandungsbereiche verhindern dabei ein Kollabieren oder Zusammenfallen des Schlauches.

Der erfindungsgemäße Schlauch ist, insbesondere unter Verwendung der oben beschriebenen Herstellungsverfahren ausge sprochen kostengünstig herstellbar, so daß insbesondere eine Anwendung als Einmalartikel möglich ist. Ein weiterer Vorteil des Schlauches besteht darin, daß dieser insgesamt nur aus Kunststoffmaterial besteht, so daß die Entsorgung des Schlauches im Gegensatz zu bekannten Schläuchen mit metallischen oder textilen Verstärkungseinlagen besonders vereinfacht wird, beispielsweise dadurch, daß der Schlauch rückstandsfrei verbrennbar ist.

Ein weiterer, wesentlicher Vorteil des erfindungsgemäßen Schlauches besteht darin, daß dieser biokompatibel ist, d.h. aus einem Material gefertigt werden kann, welches keine unerwünschten Wechselwirkungen mit den jeweiligen medizinischen Flüssigkeiten aufweist.

Im folgenden wird die Erfindung anhand zweier Ausführungsbeispiele in Verbindung mit der Zeichnung beschrieben. Dabei zeigt:

Figur 1 eine schematische Schnittansicht eines erfindungsgemäßen Schlauches mit verdickten Wandungsbereichen in konzentrischer Form und

Figur 2 eine Schnittansicht, ähnlich Figur 1, eines weiteren Ausführungsbeispieles, bei welchem die verdickten Wandungsbereiche in Form einer Spirale ausgebildet sind.

Der erfindungsgemäße Schlauch 1 weist eine Schlauchwandung 3 gleichbleibender Dicke auf und ist mit einer Innenfläche 4 versehen, welche glatt, d.h. zylindrisch ausgebildet ist. Auf diese Weise kann eine Durchströmung des Schlauches ohne Beeinträchtigung als laminare Strömung erfolgen, das Auftreten von Turbulenzen wird vermieden. In einem typischen Ausführungsbeispiel weist der Schlauch einen Innendurchmesser von 4,5 mm auf, während die Dicke der Schlauchwandung 0,1 - 1 mm beträgt.

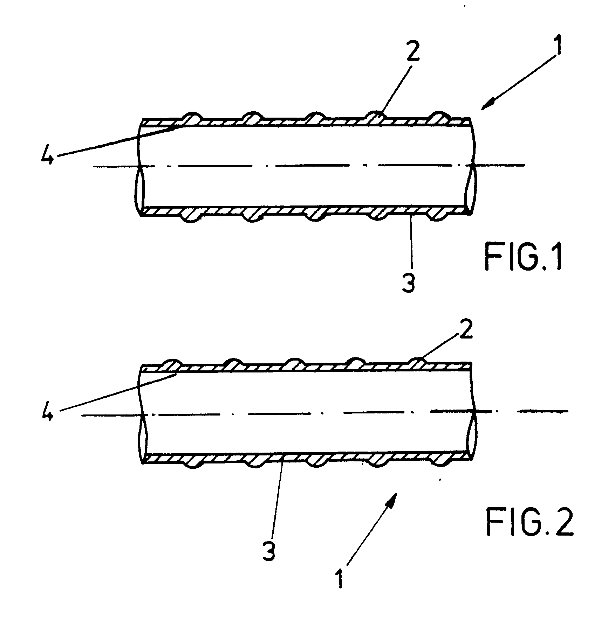

Erfindungsgemäß sind an der Schlauchwandung Wandungsbereiche 2 größerer Dicke ausgebildet, welche entweder in Form konzentrischer Ringe (sh. Figur 1) oder in Form einer Spirale (sh. Figur 2) ausgestaltet sind. Bei einem typischen Ausführungsbeispiel der oben genannten Art weisen die Wandungsbereiche, in radialer Richtung eine Dicke von 0,5 - 2 mm auf, sind somit um den Faktor 2 - 10 dicker, als die Schlauchwandung 3. In der Praxis hat es sich als besonders günstig erwiesen, die Wandungsbereiche 2 um einen Faktor 3 - 7 dicker als die Wandung des Schlauches auszugestalten.

Die Breite der Wandungsbereiche in Längsrichtung des Schlauches bzw. die Breite der freien, nicht verdickten Bereiche der Schlauchwandung 3 kann den jeweiligen Anwendungszwecken angepaßt werden, insbesondere dem Durchmesser des Schlauches und dessen Wandungsdicke. Bei Ausgestaltung der verdickten Wandungsbereiche 2 in Form einer Spirale hat es sich als günstig erwiesen, wenn die Steigungshöhe der Spirale nicht größer ist als der Durchmesser des Schlauches.

Die Erfindung ist nicht auf die gezeigten Ausführungsbeispiele beschränkt, es ergeben sich für den Fachmann vielfältige Ausgestaltungsmöglichkeiten. So ist es beispielsweise möglich, die Breite der verdickten Wandungsbereiche weiter zu erhöhen oder beispielsweise eine doppelläufige Spirale vorzusehen.

Üblicherweise werden die erfindungsgemäßen Schläuche bei einer besonders bevorzugten Herstellungsart mit Spiralen extrudiert, wobei nachzutragen wäre, daß unter der in dieser Beschreibung verwendeten Angabe "im wesentlichen ringförmig" auch "sprialförmig" zu verstehen ist. Der erfindungsgemäße Schlauch wird bevorzugter Weise aus einem transparenten Material hergestellt, das biokompatibel bzw. bioverträglich und insbesondere hierbei blutverträglich ist. Als besonders bevorzugtes Material wird ein solches verwendet, das eine Shore-Härte A 60 und mehr aufweist.

**Ansprüche**

1. Schlauch (1) zur Führung von medizinischen Flüssigkeiten
- mit einer Schlauchwandung (3); und
- mit im wesentlichen ringförmigen Verstärkungselementen (2) für die Schlauchwandung (3);
dadurch gekennzeichnet,
- daß die Schlauchwandung (3) eine gleichbleibende Dicke aufweist, und
- daß die Verstärkungselemente als Wandungsbereiche (2) größerer Dicke ausgebildet sind, welche sich um die Schlauchwandung (3) erstrecken, wobei die Wandungsbereiche (2) eine Dicke aufweisen, welche um einen Faktor 2 - 10 größer ist, als die Dicke der nicht verdickten Schlauchwandung (3).

2. Schlauch nach Anspruch 1, dadurch gekennzeichnet, daß die Wandungsbereiche (2) in Form konzentrischer Ringe ausgebildet sind.

3. Schlauch nach Anspruch 1, dadurch gekennzeichnet, daß die Wandungsbereiche (2) in Form einer Spirale ausgebildet sind.

4. Schlauch nach einem der Ansprüche 1 bis 3, dadurch gekennzeichnet, daß der Schlauch (1) eine glatte zylindrischer Innenfläche (4) aufweist.

5. Schlauch nach einem der Ansprüche 3 oder 4, dadurch gekennzeichnet, daß die Steigungshöhe der Spirale kleiner oder gleich dem Durchmesser des Schlauches (1) ist.

6. Schlauch nach einem der Ansprüche 1 bis 5, dadurch gekennzeichnet, daß die Wandungsbereiche (2) aus dem gleichen Material wie der Schlauch (1) gefertigt sind.

7. Schlauch nach einem der Ansprüche 1 bis 6, dadurch gekennzeichnet, daß die Wandungsbereiche (2) aus einem unterschiedlichen Material wie der Schlauch (1) gefertigt sind.

8. Schlauch nach einem der Ansprüche 1 bis 7, dadurch gekennzeichnet, daß der Schlauch (1) aus einem thermoplastischen Material besteht.

9. Schlauch nach einem der Ansprüche 1 bis 8, dadurch gekennzeichnet, daß der Schlauch (1) aus einem weichmacherfreien Material besteht.

10. Verfahren zur Herstellung eines Schlauches nach einem der Ansprüche 1 bis 9, dadurch gekennzeichnet, daß der Schlauch (1) mittels eines Extruders unter gleichzeitiger Ausbildung der verdickten Wandungsbereiche (2) hergestellt ist.

11. Verfahren zur Herstellung eines Schlauches nach einem der Ansprüche 1 bis 9, dadurch gekennzeichnet, daß die verdickten Wandungsbereiche (2) auf einen Schlauch (1) gleichbleibender Wanddicke aufgebracht werden.

FIG.1

FIG.2